# EUROPEAN PATENT SPECIFICATION

(11) **EP 3 758 611 B1**
(45) Date of publication and mention of the grant of the patent: **24.07.2024**
(21) Application number: 19706733.3
(22) Date of filing: 25.02.2019
(51) Int. Cl.: A61B 8/08, G01S 15/89, G01S 7/52

(54) **ULTRASOUND APPARATUS TO GUIDE INJECTION OF NON-CYTOTOXIC PROTEASE**
ULTRASCHALLGERÄT ZUR FÜHRUNG DER INJEKTION VON NICHTTOXISCHER PROTEASE
APPAREIL À ULTRASON POUR GUIDER L'INJECTION DE PROTÉASE NON CYTOTOXIQUE

(30) Priority: 26.02.2018 EP 18158702
(43) Date of publication of application: 06.01.2021
(73) Proprietor: Ipsen Biopharm Limited, Slough, Berkshire SL1 3XE (GB)
(72) Inventor: EVANS, Steve, Slough, Berkshire SL1 3XE (GB)
(74) Representative: White, Andrew John
(86) International application number: PCT/GB2019/050506
(87) International publication number: WO 2019/162696

(56) References cited:
- US-A1- 2015 165 243
- US-A1- 2016 128 668

## Description

### Field of the invention

The present disclosure relates to a method and apparatus for guiding the treatment of non-cytotoxic proteases, and in particular to a medical or surgical apparatus, computer program and method for guiding the injection of at least one non-cytotoxic protease.

### Background

Cytotoxic proteins act by killing their natural target cells. This group of toxins is exemplified *inter alia* by plant toxins such as ricin, and abrin, and by bacterial toxins such as diphtheria toxin, and Pseudomonas exotoxin A. Cytotoxic toxins typically kill their target cells by inhibiting the cellular process of protein synthesis. This class of protein includes re-targeted cytotoxic proteins in which the natural binding ability of the protein has been modified by the introduction of a binding ligand (also known as a Targeting Moiety), thereby conferring new target cell binding properties on the modified protein.

In contrast, non-cytotoxic proteins act on target cells by incapacitating cellular function. Importantly, non-cytotoxic toxins do not kill the target cells upon which they act. Some of the best known examples of non-cytotoxic proteases include clostridial neurotoxins (e.g. botulinum neurotoxin, which is marketed under names such as Dysport^{™}, Neurobloc^{™}, and Botox^{™}), IgA proteases (see, for example, WO99/032272), and antarease proteases (see, for example, WO2011/022357). Non-cytotoxic proteases act by proteolytically-cleaving and thus inactivating intracellular transport proteins known as SNARE proteins (e.g. SNAP-25, VAMP, or Syntaxin) - see Gerald K (2002) "Cell and Molecular Biology" (4th edition) John Wiley & Sons, Inc. The acronym SNARE derives from the term **S**oluble **N**SF **A**ttachment **RE**ceptor, where NSF means **N**-ethylmaleimide-**S**ensitive **F**actor. SNARE proteins are essential components of the vesicular secretion process in eukaryotic cells. Thus, non-cytotoxic proteases act by suppressing cellular secretion. This class of protein includes re-targeted non-cytotoxic proteins in which the natural binding ability of the protein has been modified by the introduction of a binding ligand (also known as a Targeting Moiety), thereby conferring new target cell binding properties on the modified protein. Applicant has pioneered the technology relating to the re-targeting of non-cytotoxic proteases, which dates back to the 1990s (see, for example, WO 94/21300, WO 96/33273 and WO 98/07864). Said re-targeted proteins are referred to (throughout the literature and scientific community) as Targeted Secretion Inhibitors (TSIs) - reference to TSIs includes structural equivalents such as those described in WO 20111/018665.

Non-cytotoxic proteases may be employed in their native or substantially native forms (i.e. as holotoxins, such as BOTOX^{™}), in which case targeting of the proteases to specific cell-types is reliant on (i) localised administration of the protease and/ or (ii) the inherent binding ability of the native protease. Alternatively, non-cytotoxic proteases may be employed in a re-targeted form in which the native protease is modified to include an exogenous ligand known as a Targeting Moiety (TM). The TM is selected to provide binding specificity for a desired target cell, and, as part of the re-targeting process, the native binding portion of the non-cytotoxic protease may be removed. Re-targeting technology has patent filings dating back to the early 1990s and include: EP-B-0689459; EP-B-0939818; US 6,461,617; US 7,192,596; EP-B-0826051; US 5,989,545; US 6,395,513; US 6,962,703; EP-B-0996468; US 7,052,702; EP-B-1107794; and US 6632440.

In view of the ubiquitous nature of SNARE proteins, non-cytotoxic proteases have been successfully employed in a plethora of therapies.

By way of example, we refer to William J. Lipham, Cosmetic and Clinical Applications of Botulinum Toxin (Slack, Inc., 2004), which describes the use of Clostridial toxins, such as botulinum neurotoxins (BoNTs), BoNT/A, BoNT/B, BoNT/C1, BoNT/D, BoNT/E, BoNT/F and BoNT/G, and tetanus neurotoxin (TeNT), to inhibit neuronal transmission in a wide variety of therapeutic and cosmetic applications - as an example, BOTOX^{™} is currently approved as a therapeutic for the following indications: achalasia, adult spasticity, anal fissure, back pain, blepharospasm, bruxism, cervical dystonia, essential tremor, glabellar lines or hyperkinetic facial lines, headache, hemifacial spasm, hyperactivity of bladder, hyperhidrosis, juvenile cerebral palsy, multiple sclerosis, myoclonic disorders, nasal labial lines, spasmodic dysphonia, strabismus and VII nerve disorder. In addition, Clostridial toxin therapies are described for treating neuromuscular disorders (see US 6,872,397; for treating uterine disorders (see US2004/0175399); for treating ulcers and gastroesophageal reflux disease (see US2004/0086531); for treating dystonia (see US 6,319,505); for treating eye disorders (see US2004/0234532); for treating blepharospasm (see US2004/0151740); for treating strabismus (see US2004/0126396); for treating pain (see US 6,869,610, US 6,641,820, US 6,464,986, US 6,113,915); for treating fibromyalgia (see US 6,623,742, US2004/0062776); for treating lower back pain (see US2004/0037852); for treating muscle injuries (see US 6,423,319); for treating sinus headache (see US 6,838,434); for treating tension headache (see US 6,776,992); for treating headache (see US 6,458,365); for reduction of migraine headache pain (see US 5,714,469); for treating cardiovascular diseases (see US 6,767,544); for treating neurological disorders such as Parkinson's disease (see US 6,620,415, US 6,306,403); for treating neuropsychiatric disorders (see US2004/0180061, US2003/0211121); for treating endocrine disorders (see US 6,827,931); for treating thyroid disorders (see US 6,740,321); for treating a cholinergic influenced sweat Gland (see US 6,683,049); for treating diabetes (see US 6,337,075, US 6,416,765); for treating a pancreatic disorder (see US 6,261,572, US 6,143,306); for treating cancers such as bone tumors (see US 6,565,870, US 6,368,605, US 6,139,845, US2005/0031648); for treating otic disorders (see US 6,358,926, US 6,265,379); for treating autonomic disorders such as gastrointestinal muscle disorders and other smooth muscle dysfunction (see US 5,437,291); for treatment of skin lesions associated with cutaneous cell-proliferative disorders (see US 5,670,484); for management of neurogenic inflammatory disorders (see US 6,063,768); for reducing hair loss and stimulating hair growth (see US 6,299,893); for treating downturned mouth (see US 6,358,917); for reducing appetite (see US2004/40253274); for dental therapies and procedures (see US2004/0115139; for treating neuromuscular disorders and c (see US2002/0010138); for treating various disorders and conditions and associated pain (see US2004/0013692) for treating pain (see WO96/33274); for treating conditions resulting from mucus hypersecretion such as asthma and COPD (see WO00/10598); for treating non-neuronal conditions such as inflammation, endocrine conditions, exocrine conditions, immunological conditions, cardiovascular conditions, bone conditions (see WO01/21213).

The use of non-cytotoxic proteases such as clostridial neurotoxins (e.g. BoNTs and TeNT) in therapeutic and cosmetic treatments of humans and other mammals is anticipated to expand to an ever-widening range of diseases and ailments that can benefit from the properties of these toxins.

Generally, therapeutic administration of a non-cytotoxic protease, including native botulinum neurotoxin clinical products, is well tolerated. However, administration in some applications can be challenging because of the larger doses required to achieve a beneficial effect. Larger doses can increase the likelihood that the protease may move, for example, through the interstitial fluids and the circulatory systems (such as the cardiovascular system and the lymphatic system) of the body, resulting in undesirable dispersal of the protease to areas not targeted for treatment. Said dispersal can lead to undesirable side effects, such as inhibition of cellular secretion in cells not targeted for treatment (e.g. inhibition of neurotransmitter release in neurons not targeted for treatment, or paralysis of a muscle not targeted for treatment). By way of specific example, a patient administered a therapeutically effective amount of a BoNT into the neck muscles for torticollis may develop dysphagia because of dispersal of the protease into the oropharynx. Similarly, a patient administered a non-cytotoxic protease to treat a neuromuscular disorder may suffer from undesirable muscle tissue inactivation due to dispersal of the protease into the muscle.

In common with any other drug substances, a therapeutic dosing range exists which identifies the lower and upper limits of safe, effective therapy. Often, the upper limit is determined by the increasing significance of off-target effects that lead to undesirable (e.g. potentially harmful) side-effects of drug treatment. In the case of non-cytotoxic proteases (notably BoNT), this could lead to the paralysis of cellular secretion in off-target cells, which, in turn, could be fatal.

The growing clinical, therapeutic and cosmetic use of non-cytotoxic proteases in therapies requiring larger doses places an ever-increasing requirement on the part of the pharmaceutical industry to develop means for minimising off-target effects, such that the therapeutic dose range can be increased and the patients thus provided with increased doses which will, in turn, lead to increased efficacy of treatment.

There is therefore a need in the art for addressing undesirable, off-site targeting effects.

Embodiments of the disclosure may address the above-mentioned problems. Document US2016/128668 relates to systems and methods for real-time highlighting of instruments in images using ultrasound probes.

### Summary of the invention

The invention is defined by the independent claims 1, 4, 10 and 11. Optional features are set out in the dependent claims. Any disclosed methods are merely exemplary and do not fall within the scope of the present invention.

In a first aspect there is provided a medical or surgical apparatus for guiding the injection of at least one non-cytotoxic protease, the apparatus comprising:
an ultrasound imaging handheld scanner; and
a computer program comprising instructions which, when the program is executed by a computer, cause the computer to run an ultrasound imaging processing application;
wherein the ultrasound imaging handheld scanner comprises:
   at least one ultrasound transducer for transmitting and receiving ultrasound energy;
   a processor coupled to the at least one ultrasound transducer and configured to receive ultrasound energy signals from the at least one ultrasound transducer; and
   an interface coupled to the processor and configured to send data to the computer running the ultrasound imaging processing application, wherein the data is based on the received ultrasound energy signal from the at least one ultrasound transducer; and
wherein the ultrasound imaging processing application is configured to reference a database of treatment sites and dosage regimes for at least one non-cytotoxic protease, and is configured to provide treatment guidance to a user of the medical or surgical apparatus for guiding treatment based on the received data from the ultrasound imaging handheld scanner and the database of treatment sites and dosage regimes for the at least one non-cytotoxic protease.

The treatment site may correspond to any anatomical site and/or in any tissue type. For example the treatment site may be a treatment site in at least one of: muscle, organs (such as bladder), nerves (for the purpose of a nerve block) or may be percutaneous (for example for cosmetic applications).

The medical or surgical apparatus may comprise a controlled delivery device coupled to the ultrasound imaging handheld scanner, wherein the controlled delivery device is configured to deliver at least one controlled dose of at least one non-cytotoxic protease to a patient being imaged by the ultrasound imaging handheld scanner based on a location relative to the patient determined based on the received ultrasound energy signals.

Additionally or alternatively, the medical or surgical apparatus may comprise a guide for receiving at least a portion of an injection device, such as a needle, wherein the guide is coupled to the ultrasound imaging handheld scanner, and wherein the computer running the ultrasound imaging processing application is configured to display information to the user for enabling the user to position the guide at a determined position relative to the patient based on the database of treatment sites and dosage regimes for the at least one non-cytotoxic protease, so that the user can inject the non-cytotoxic protease into the patient at the correct treatment site for the selected non-cytotoxic protease being injected.

In some examples, a computer such as the computer running the ultrasound imaging processing application, or a separate computer (for example a handheld device such as a smartphone) that may or may not be in communication with the ultrasound imaging processing application running on another computer, may be configured to calculate and determine a dosage regime for a patient. For example, the computer may comprise a dosage application that is configured to receive a series of inputs from a user and in response display information to the user regarding the treatment sites and dosage regimes for the patient to assist in the delivery of treatment to a patient.

For example, the dosage application may be configured to receive information regarding the patient (such as age, weight, region and/or part of the anatomy (for example muscle type)) and determine a recommended treatment regime and/or dosage range for the patient. The recommended dosage range may be determined, for example, on the basis of the information regarding the patient. The recommended dosage range may be calculated per region and/or part of the anatomy (for example, muscle type) based on a determined total dose for the patient. The dosage application may provide a warning indication to the user if a dose outside of the recommended dosage range is selected.

The dosage application may also be configured to assist in preparing samples for injection. For example, the dosage application may be configured to receive inputs regarding the type and concentration of treatment, and calculate the necessary volume of treatment to be injected based on a determined or selected dose for a particular treatment site, which is then displayed to the user. Calculating the necessary volume of treatment to be injected may comprise calculating the number of vials of a particular treatment that are necessary to deliver the desired treatment at a treatment site.

In another aspect there is provided a medical or surgical apparatus for guiding the injection of at least on non-cytotoxic protease, the apparatus comprising:
an ultrasound imaging handheld scanner; and
a controlled delivery device coupled to the ultrasound imaging handheld scanner;
wherein the ultrasound imaging handheld scanner comprises:
   at least one ultrasound transducer for transmitting and receiving ultrasound energy;
   a processor coupled to the at least one ultrasound transducer and configured to receive ultrasound energy signals from the at least one ultrasound transducer; and
wherein the controlled delivery device is configured to deliver a controlled dose of at least one non-cytotoxic protease to a patient being imaged by the ultrasound imaging handheld scanner based on a location relative to the patient determined based on the received ultrasound energy signal.

The medical or surgical apparatus may comprise an interface coupled to the processor and configured to send data to a computer running an ultrasound imaging processing application, wherein the data is based on the received ultrasound energy signals from the at least one ultrasound transducer. The ultrasound imaging processing application may comprise a database of treatment sites and dosage regimes for at least one non-cytotoxic protease, and may be configured to provide treatment guidance to a user of the medical or surgical apparatus for guiding treatment based on the received data from the ultrasound imaging handheld scanner and the database of treatment sites and dosage regimes for the at least one non-cytotoxic protease.

The treatment guidance may comprise an indication of at least one of:
(i) where to inject the non-cytotoxic protease relative to the patient being scanned; and
(ii) a quantity of non-cytotoxic protease to inject.

The treatment guidance may additionally or alternatively comprise at least one of: a recommended dose range for a selected region of the anatomy (such as a tissue type, for example a muscle type), a recommended total patient dose, guidance as to how to prepare the treatment for injection such as the concentration and volume of treatment needed, and the number of vials or syringes needed to deliver the treatment.

The treatment guidance may comprise an indication of where to inject the non-cytotoxic protease relative to the patient being scanned, and the indication may be overlaid on a graphical representation of the patient being scanned based on the data received from the ultrasound imaging handheld scanner.

The ultrasound imaging processing application may be configured to determine the position of a controlled delivery device coupled to the ultrasound imaging handheld device, or an injection device positioned in a guide coupled to the ultrasound imaging handheld device, relative to the patient based on the received data from the ultrasound imaging handheld device. The treatment guidance may comprise an indication to the user of how to position the ultrasound imaging handheld scanner with respect to the patient so that the non-cytotoxic protease can be injected from the controlled delivery device or injection device positioned in the guide at the correct treatment site.

The ultrasound imaging processing application may be configured to process the data received from the ultrasound imaging handheld device to provide a graphical representation of the patient being scanned to the user.

The processor of the ultrasound imaging handheld scanner may be configured to process the received ultrasound energy signals from the at least one ultrasound transducer to provide data signals comprising information indicative of a graphical representation of the patient being scanned.

In another aspect there is provided a medical or surgical kit comprising:
an ultrasound imaging handheld scanner,
an injectable device comprising a selected quantity of at least one non-cytotoxic protease; and
a computer program comprising instructions which, when the program is executed by a computer, cause the computer to run an ultrasound imaging processing application;
wherein the ultrasound imaging processing application is configured to reference a database of treatment sites and dosage regimes for at least one non-cytotoxic protease, and is configured to provide treatment guidance to a user of the medical or surgical apparatus for guiding treatment based on the received data from the ultrasound imaging handheld scanner and the database of treatment sites and dosage regimes for the at least one non-cytotoxic protease, for enabling the user to inject the correct quantity of non-cytotoxic protease at the correct treatment site.

The kit may further comprise a controlled delivery device coupled to the ultrasound imaging handheld scanner, wherein the controlled delivery device is configured to deliver a controlled dose of at least one non-cytotoxic protease to a patient being imaged by the ultrasound imaging handheld scanner based on a location relative to the patient determined based on the received ultrasound energy signal.

Additionally or alternatively, the kit may further comprise a guide for receiving at least a portion of an injection device, such as a needle, wherein the guide is coupled to the ultrasound imaging handheld scanner. The computer running the ultrasound imaging processing application may be configured to display information to the user for enabling the user to position the guide at a determined position relative to the patient based on the database of treatment sites and dosage regimes for the at least one non-cytotoxic protease, so that the user can inⱼect the non-cytotoxic protease into the patient at the correct treatment site for the selected non-cytotoxic protease being injected.

In another aspect there is provided a computer program comprising instructions which, when the program is executed by a computer, cause the computer to run an ultrasound imaging processing application, wherein the ultrasound imaging processing application is configured to:
receive and process data obtained from an ultrasound imaging handheld scanner, reference a database of treatment sites and dosage regimes for at least one non-cytotoxic protease; and
provide treatment guidance to a user of the medical or surgical apparatus for guiding treatment based on the received data from the ultrasound transducer and the database of treatment sites and dosage regimes for the at least one non-cytotoxic protease, for enabling the user to inject the correct quantity of non-cytotoxic protease at the correct treatment site.

The ultrasound imaging processing application may be configured to process the data received from the ultrasound imaging handheld device to provide a graphical representation of the patient being scanned to the user.

The treatment guidance may comprise an indication of where to inject the non-cytotoxic protease relative to the patient being scanned. The indication may be overlaid on a graphical representation of the patient being scanned based on the data received from the ultrasound imaging handheld scanner.

The ultrasound imaging processing application may be configured to determine the position of at least one of: (i) a controlled delivery device coupled to the handheld ultrasound imaging scanner, and (ii) an injection device positioned in a guide and coupled to the handheld ultrasound imaging scanner, relative to the patient based on the received data from the ultrasound imaging handheld device. The treatment guidance may comprise an indication to the user of how to position the ultrasound imaging handheld scanner with respect to the patient so that the non-cytotoxic protease can be injected from the controlled delivery device or injection device positioned in the guide at the correct treatment site.

Furthermore, a method of administering a controlled dose of non-cytotoxic protease to a patient is disclosed, the method comprising:
scanning a region of a patient's anatomy with an ultrasound imaging handheld scanner to obtain a graphical representation of the region of the patient's anatomy being scanned;
referencing a database of treatment sites and dosage regimes for the non-cytotoxic protease being administered;
determining at least one of:
   (i) the correct location to inject the non-cytotoxic protease being administered based on the graphical representation of the region of the patient's anatomy and the reference with the database of treatment sites and dosage regimes; and
   (ii) the correct dose of the non-cytotoxic protease to administer based on the graphical representation of the region of the patient's anatomy and the reference with the database of treatment sites and dosage regimes;
administering a controlled dose of the non-cytotoxic protease at the correct location based on the determination.

Determining the correct dose of the non-cytotoxic protease to administer based on the graphical representation of the region of the patient's anatomy and the reference with the database of treatment sites and dosage regimes may comprise determining the position of at least one of (i) a controlled delivery device coupled to the handheld ultrasound imaging scanner relative to the patient, and (ii) an injection device positioned in a guide and coupled to the handheld ultrasound imaging scanner relative to the patient.

### Drawings

Embodiments of the disclosure will now be described, by way of example only, with reference to the accompanying drawings, in which:
Fig. 1 shows an example medical or surgical apparatus for guiding the injection of non-cytotoxic proteases;
Fig. 2 shows another example medical or surgical apparatus for guiding the injection of non-cytotoxic proteases;
Fig. 3 shows another example medical or surgical apparatus for guiding the injection of non-cytotoxic proteases;
Fig. 4 shows an example flow-chart for use in administering a dose of non-cytotoxic protease to a patient;
Fig. 5 shows another example medical or surgical apparatus for guiding the injection of non-cytotoxic proteases;
Fig. 6 shows two screenshots of an example computer program for use with the medical or surgical apparatus of any of Figs. 1 to 3 and 5;
Fig. 7 shows two further screenshots of the example computer program of Fig. 6 for use with the medical or surgical apparatus of any of Figs. 1 to 3 and 5; and
Fig. 8 shows another screenshot of the example computer program of Figs. 6 and 7 for use with the medical or surgical apparatus of any of Figs. 1 to 3 and 5.

### Specific description

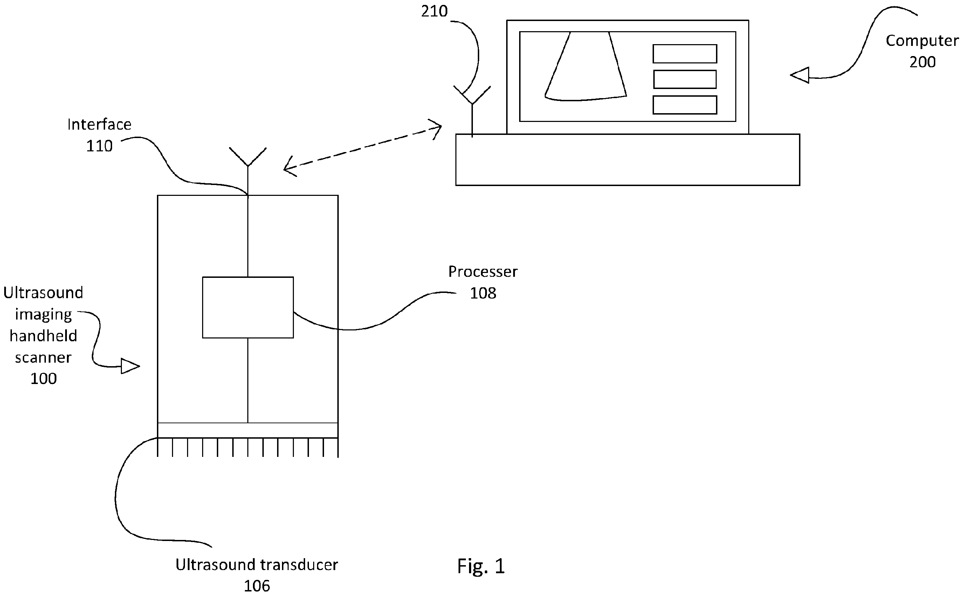

Fig. 1 shows an example medical or surgical apparatus for guiding the treatment, for example the injection, of at least one non-cytotoxic protease. The at least one non-cytotoxic protease may be supplied as part of a medical composition, for example at least one polypeptide. In some examples, the medical composition (such as a polypeptide) comprises:
(i) at least one non-cytotoxic protease, which protease is capable of cleaving a SNARE protein in a cell - for example, the non-cytotoxic protease may be a clostridial neurotoxin such as BoNT and/or TeNT;
(ii) at least one Targeting Moiety (TM) that is capable of binding to a Binding Site on a cell, which Binding Site is capable of undergoing endocytosis to be incorporated into an endosome within the cell, and wherein said cell expresses said SNARE protein; and
(iii) at least one translocation domain that is capable of translocating the protease from within an endosome, across the endosomal membrane and into the cytosol of the cell.

Fig. 1 shows an ultrasound imaging handheld scanner 100 comprising an ultrasound transducer 106, a processor 108 and an interface 110, such as a wired or wireless interface, for example a Bluetooth^{®} interface. The ultrasound imaging handheld scanner 100 may also be known as an ultrasound wand. The processor 108 is coupled to the ultrasound transducer 106 and the interface 106. Although only one ultrasound transducer 106 is shown in Fig. 1, it will be understood that in other examples there may be a plurality of ultrasound transducers 106, for example an array of ultrasound transducers 106.

Also shown in Fig. 1 is a computer 200 operating an ultrasound imaging processing application. It will be understood that the medical or surgical apparatus may comprise a computer program comprising instructions which, when run on the computer, cause the computer to run the ultrasound imaging processing application. The computer 200 comprises an interface 210, such as a wired or wireless interface, for example a Bluetooth^{®} interface. It will be understood that in some examples the computer 200 may be a desktop or laptop computer, or in other examples may be a portable electronic device, for example a handheld device such as a tablet or mobile/cell phone.

In use, the ultrasound transducer 106 is operable to transmit and receive ultrasound energy for the purpose of ultrasound scanning, for example to create a graphical representation of a region of a patient, such as a sonogram. The processor 108 is configured to receive the signals from the ultrasound transducer 106.

In some examples, the processor 108 is configured to process the received ultrasound energy signals from the at least one ultrasound transducer to provide data signals comprising information indicative of a graphical representation of the patient being scanned. However, in other examples, minimal (if any) processing is performed by the processor 108. The interface 110 is configured to receive data from the processor 108 and send data based on the received ultrasound energy signal from the at least one ultrasound transducer 106 to the computer.

The interface 210 of the computer 200 is configured to receive signals comprising the data from the ultrasound imaging handheld scanner 100. The ultrasound imaging processing application running on the computer 200 is configured to reference a database of treatment sites and dosage regimes for at least one non-cytotoxic protease, and is configured to provide treatment guidance to a user of the medical or surgical apparatus for guiding treatment based on the received data from the ultrasound imaging handheld scanner and the database of treatment sites and dosage regimes for the at least one non-cytotoxic protease. The database could be a local database (for example, stored on the computer 200) or could be a remote database (for example, the database could be a cloud-based database, for example accessed via the Internet).

The treatment guidance may comprise an indication of at least one of:
(i) where to inject the, or a plurality of, non-cytotoxic protease(s) relative to the patient being scanned; and
(ii) a quantity of each of the non-cytotoxic protease(s) to inject.

In some examples, the ultrasound imaging processing application is configured to process the data received from the ultrasound imaging handheld device 100 to provide a graphical representation of the patient being scanned to the user, for example in the form of a sonogram. In such examples, the treatment guidance may comprise an indication of where to inject the non-cytotoxic protease relative to the patient being scanned, and the indication may be overlaid on a graphical representation of the patient being scanned based on the data received from the ultrasound imaging handheld scanner. For example, the treatment guidance may indicate on a sonogram where the non-cytotoxic protease should be injected to have increased efficacy and minimise off-target effects.

In some examples, processing the data received from the ultrasound imaging handheld device 100 to provide a graphical representation of the patient being scanned to the user may comprise performing image recognition on a graphical representation of the patient being scanned. For example, the ultrasound imaging processing application may comprise algorithms for performing image recognition. For example, the ultrasound imaging processing application may be configured to detect which region and/or part of the anatomy is being scanned and to use this information when referencing the database for determining treatment guidance.

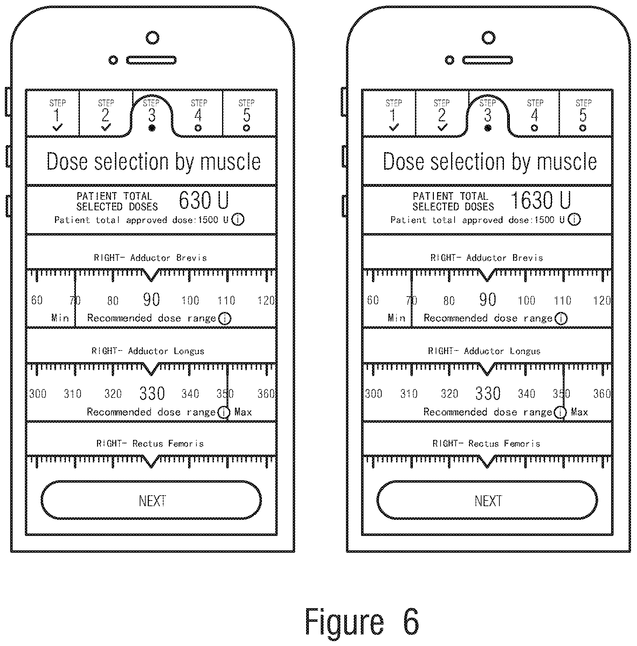

In some examples, the image processing application may additionally or alternatively comprise an interface for enabling a user to enter patient-specific details, for example regarding the patient's weight, age, symptoms, region and/or part of the anatomy being imaged, for example to aid in the image recognition process and/or for use in performing the reference with the database for determining treatment guidance, for example as described below with reference to Figs. 6 to 8.

In some examples, the image processing application may be configured to receive patient-specific data from the patient, and make a determination of the treatment site and/or treatment dose based on the received patient-specific data (as well as, for example, the database of treatment sites and dosage regimes). The treatment dose may be a dose for a particular treatment site, and/or a total dose for a patient. The treatment dose may also comprise a preferred or recommended dose range. The treatment dose may be specific to a particular non-cytotoxic protease, or may be a dose determined irrespective of non-cytotoxic protease type (i.e. a dose for all non-cytotoxic proteases). Such examples may provide for "personalised medicine" where the treatment can be tailored for each specific patient. The patient-specific data may comprise at least one of: patient's age, patient's weight, patient's symptoms, tissue type to be treated, non-cytotoxic protease being used, degree of disability, and time since injury/disabilitating event. For example, the patient's degree of disability, for example degree of spasticity, may be used in determining the treatment dose and/or treatment site. For example, the patient may be given a score based on the severity of their symptoms to determine their degree of disability, and may be used in determining the treatment dose and/or treatment site. Additionally or alternatively, the time since a disabilitating event, such as a stroke, may be used in determining the treatment dose and/or treatment site. For example, it may be envisaged that the treatment dose may decrease as a function of increasing time since the disabilitating event.

In some examples, the medical or surgical apparatus may comprise an injectable device comprising a selected quantity of at least one non-cytotoxic protease. For example, the medical or surgical apparatus may be provided as part of a kit, comprising the ultrasound imaging handheld scanner, a computer program comprising instructions which, when the program is executed by a computer, cause the computer to run the ultrasound imaging processing application, and the injectable device comprising a selected quantity of at least one non-cytotoxic protease. The injectable device may be a syringe, or may be a vial or other storage medium for holding a selected quantity of the non-cytotoxic protease. A selected quantity may comprise at least one of a selected volume and a selected concentration.

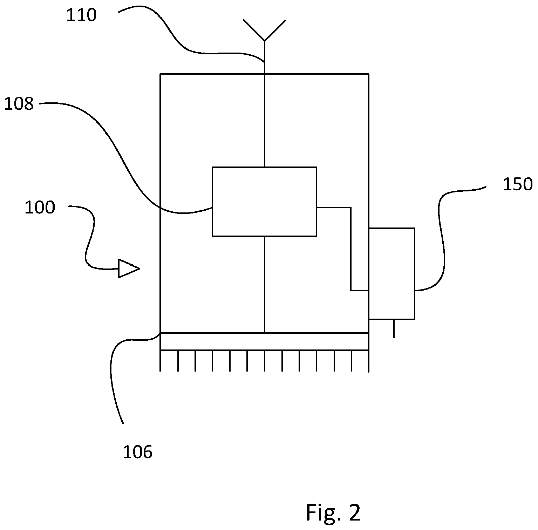

In some examples, such as the example shown in Fig. 2, the ultrasound imaging handheld scanner 100 may comprise a controlled delivery device 150 coupled to the ultrasound imaging handheld scanner 100. The controlled delivery device 150 may be configured to deliver a controlled dose of at least one non-cytotoxic protease to a patient being imaged by the ultrasound imaging handheld scanner 100, for example based on a location relative to the patient. The location relative to the patient may be determined based on ultrasound energy signals received from the ultrasound imaging handheld scanner 100. The ultrasound imaging processing application may be configured to operate the controlled delivery device 150 to administer (for example, inject) a selected quantity of non-cytotoxic protease to the patient. The ultrasound imaging processing application may be configured to operate the controlled delivery device 150 based on a determination of the correct treatment dose and treatment site made based on a reference with the database of treatment sites and dosage regimes for the selected non-cytotoxic protease in the controlled delivery device 150.

In some examples, the ultrasound imaging processing application is configured to determine the position of the controlled delivery device 150 relative to the patient based on the received data from the ultrasound imaging handheld device 100. The treatment guidance may comprise an indication to the user of how to position the ultrasound imaging handheld scanner 100 with respect to the patient so that the non-cytotoxic protease can be injected from the controlled delivery device 150 at the correct treatment site.

For example, the controlled delivery device 150 may be attached to an outer surface of the housing/casing of the ultrasound imaging handheld scanner 100. The position of the controlled delivery device 150 relative to the ultrasound transducer 106 and/or the ultrasound imaging handheld scanner 100 may be known. For example, the ultrasound imaging processing application may be programmed with information relating to the position of the controlled delivery device 150 relative to the ultrasound transducer such that the position of the controlled delivery device 150 is known. For example, the position of controlled delivery device 150 can be shown on a graphical representation of the patient's anatomy being imaged by the ultrasound imaging handheld scanner 100.

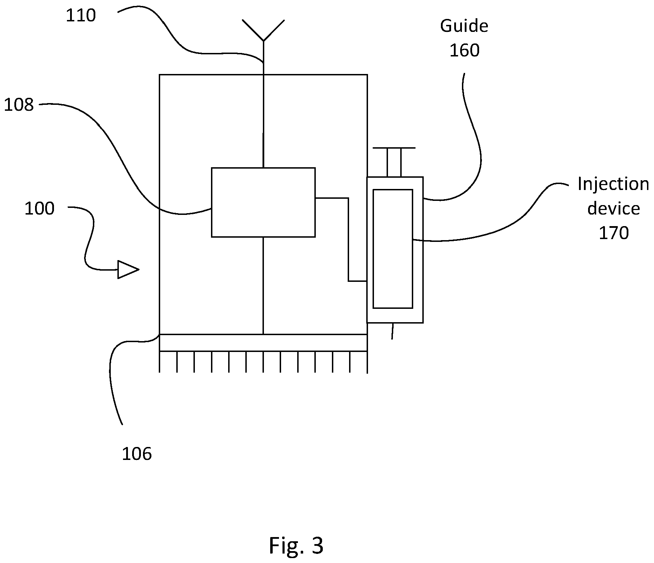

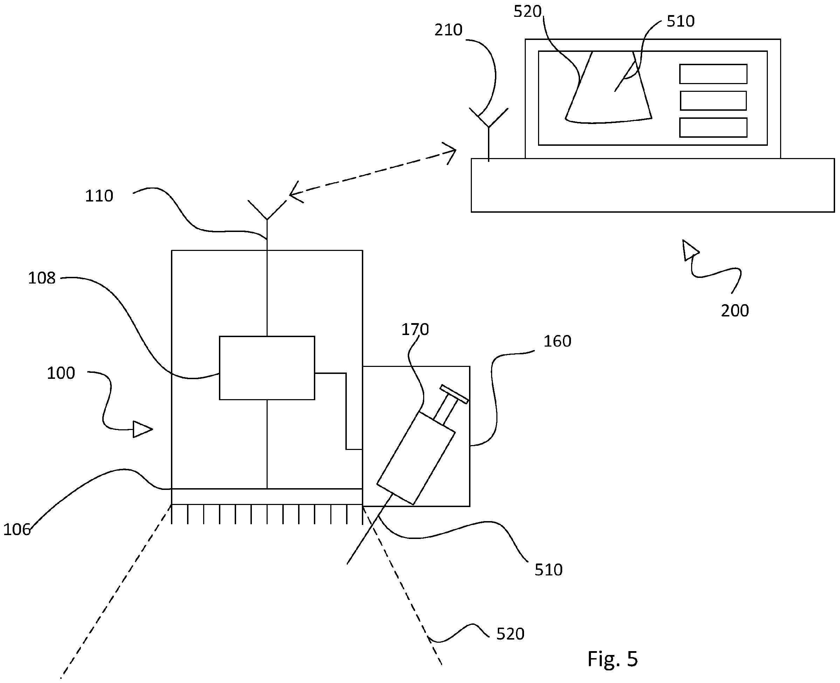

In some examples, such as the examples shown in Figs. 3 and 5, the ultrasound imaging handheld scanner 100 may comprise a guide 160 for receiving at least a portion of an injection device 170, such as a syringe. The guide 160 may be coupled to the ultrasound imaging handheld scanner 100, and the computer running the ultrasound imaging processing application may be configured to display information to the user for enabling the user to position the guide at a determined position relative to the patient based on the database of treatment sites and dosage regimes for the at least one non-cytotoxic protease, so that the user can inject the non-cytotoxic protease into the patient at the correct treatment site for the selected non-cytotoxic protease being injected from the injection device 170.

For example, the guide 160 may be attached to an outer surface of the housing/casing of the ultrasound imaging handheld scanner 100. The guide160 may comprise a slot or other means for receiving at least a portion of an injection device, such as a needle. The position of the guide 160 relative to the ultrasound transducer 106 and/or the ultrasound imaging handheld scanner 100 may be known. For example, the ultrasound imaging processing application may be programmed with information relating to the position of the guide 160 relative to the ultrasound transducer such that the position of an injectable device such as a needle inserted into the guide 160 is known. For example, the position of a needle located in the guide 160 can be shown on a graphical representation of the patient's anatomy being imaged by the ultrasound imaging handheld scanner 100.

In some examples, the ultrasound imaging processing application is configured to determine the position of an injection device 170 positioned in the guide 160, relative to the patient based on the received data from the ultrasound imaging handheld scanner 100. In such examples, the treatment guidance may comprise an indication to the user of how to position the ultrasound imaging handheld scanner 100 with respect to the patient so that the non-cytotoxic protease can be injected from the injection device 170 positioned in the guide 160 at the correct treatment site.

In some examples, such as the example shown in Fig. 5, the guide 160 will be configured to direct the injection device 170 at least partially into a field of view of the ultrasound transducer(s) 106. For example, the guide 160 will be configured so that when an injection device 170, such as a syringe, is inserted into the guide 160, it will at least partially pass into the field of view of the ultrasound transducer(s) 106. For example, as shown in Fig. 5, the needle 510 of the syringe 170 can be seen passing into the field of view 520 of the ultrasound transducer 106. In this example the ultrasound imaging processing application running on the computer 200 displays a sonogram obtained from the ultrasound transducer 106. Because the needle 510 extends at least partially into the field of view of the ultrasound transducer 106, the needle 510 can be seen in the sonogram. In this way, a user of the ultrasound imaging handheld scanner 100 can scan and identify the treatment site, introduce the syringe 170 and needle 510 into the guide 160, check the site again, advance the needle 510, check it can be seen in the field of view 520, inject the non-cytotoxic protease at the correct treatment site, withdraw the syringe 170 and check the site again.

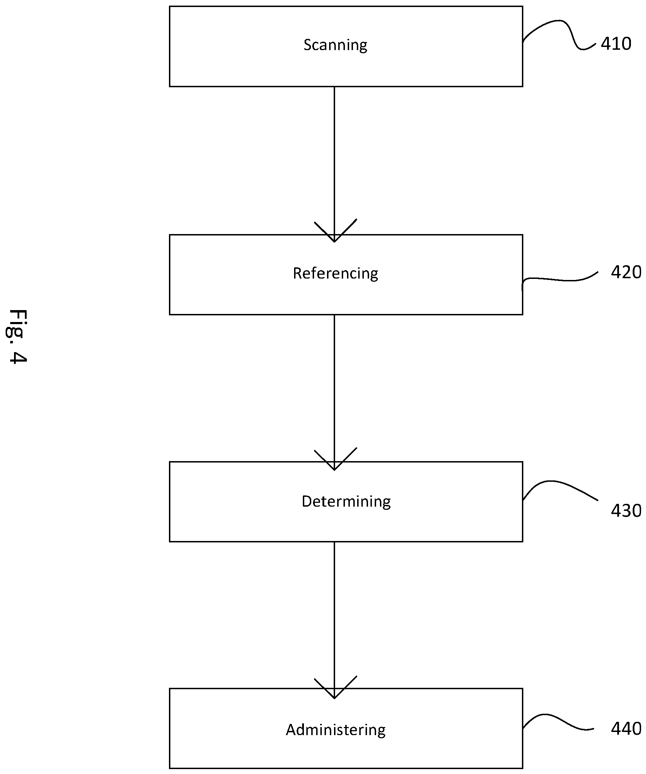

Fig. 4 shows a flow-chart for an exemplary method of administering a controlled dose of non-cytotoxic protease to a patient, for example using the apparatus and/or ultrasound imaging processing application described above with reference to Figs. 1 to 3.

The method comprises the steps of:
scanning 410 a region of a patient's anatomy with an ultrasound imaging handheld scanner to obtain a graphical representation of the region of the patient's anatomy being scanned;
referencing 420 a database of treatment sites and dosage regimes for the non-cytotoxic protease being administered;
determining 430 at least one of:
   (i) the correct location to inject the non-cytotoxic protease being administered based on the graphical representation of the region of the patient's anatomy and the reference with the database of treatment sites and dosage regimes; and
   (ii) the correct dose of the non-cytotoxic protease to administer based on the graphical representation of the region of the patient's anatomy and the reference with the database of treatment sites and dosage regimes;
administering 440 a controlled dose of the non-cytotoxic protease at the correct location based on the determination.

As described above in relation to Fig. 1, the database could be a local database (for example, stored on a computer 200) or could be a remote database (for example, the database could be a cloud-based database, for example accessed via the Internet).

In some examples, determining 430 the correct dose of the non-cytotoxic protease to administer based on the graphical representation of the region of the patient's anatomy and the reference with the database of treatment sites and dosage regimes comprises determining the position of at least one of (i) a controlled delivery device coupled to the handheld ultrasound imaging scanner relative to the patient (such as the controlled delivery device 150 described above in relation to Fig. 2), and (ii) an injection device positioned in a guide and coupled to the handheld ultrasound imaging scanner relative to the patient (such as the injection device 170 and guide 160 described above in relation to Fig. 3).

Administering 440 a controlled dose of the non-cytotoxic protease may comprise injecting a controlled dose of the non-cytotoxic protease into the patient. This may be aided, for example, via the provision of a guide 160 as illustrated in Fig. 3, or may be performed automatically/electronically, for example by the ultrasound imaging processing application, via the use of a controlled delivery device 150, as illustrated in Fig. 2.

It will be understood that in some examples, the interface 110 of the ultrasound imaging handheld scanner 100 is optional. For example, the processor 108 of the ultrasound imaging handheld scanner 100 may be configured to run the ultrasound imaging processing application. In addition, the processor 108 may be coupled to a memory that comprises the reference database of treatment sites and dosage regimes for the at least one non-cytotoxic protease.

As with the examples described above in relation to Figs. 2 and 3, in some examples the processor 108 of the ultrasound imaging handheld scanner 100 may be configured to run the ultrasound imaging processing application and may be configured to use the interface 110 to access and reference a remote database of treatment sites and dosage regimes for the at least one non-cytotoxic protease.

In some examples, the ultrasound imaging handheld scanner 100 may comprise a display or other display means to display treatment guidance to a user. For example, the ultrasound imaging handheld scanner 100 may comprise one or a plurality of lights, such as LEDs, operable to display information such an indication of an orientation in which to hold the scanner 100 and/or in which direction to move the scanner 100 for guiding treatment, such as the injection, of the non-cytotoxic protease.

In some examples, the ultrasound imaging handheld scanner 100 may comprise a display, such as an LED display, for example operable to provide a graphical representation of the region of the part of the anatomy being imaged. For example, the processor 108 of the ultrasound imaging handheld scanner 100 may be configured to process the data received from the ultrasound imaging handheld device 100 to provide a graphical representation of the patient being scanned to the user on the in-built display, for example in the form of a sonogram. It will be understood that in such examples, the user may advantageously be able to better treat a patient with a dose of non-cytotoxic protease without the need for additional computing equipment. In such examples, the treatment guidance may comprise an indication of where to inject the non-cytotoxic protease relative to the patient being scanned, and the indication may be overlaid on a graphical representation of the patient being scanned based on the data received from the ultrasound imaging handheld scanner. For example, the treatment guidance may indicate on a sonogram where the non-cytotoxic protease should be injected to have increased efficacy and minimise off-target effects.

Such examples may enable a non-skilled user of the ultrasound imaging handheld scanner 100 to self-administer treatment accurately and safely, thus avoiding the need for skilled clinical intervention (for example avoiding the need for a skilled radiographer).

In examples where the medical or surgical apparatus comprises an injectable device (such as the controlled delivery device 150 or injection device 170 described above with reference to Figs. 2 and 3) comprising a selected quantity of at least one non-cytotoxic protease, and the processor 108 of the ultrasound imaging handheld scanner 100 is configured to run the ultrasound imaging processing application, then the medical or surgical apparatus may be supplied as a kit, for example in a sealed sterile container, comprising both the ultrasound imaging handheld scanner 100 and the injectable device containing the non-cytotoxic protease. This may be advantageous not only for hygiene reasons but also because it may mean that the dosage of non-cytotoxic proteases may be better controlled. This may be because a controlled dose/quantity of non-cytotoxic protease can be supplied with a device that has been programmed (for example the processor 108 may be programmed, or a memory coupled to the processor 108 may be programmed) with information relating to at least one of (i) the concentration and (ii) the volume of non-cytotoxic protease supplied with it so that the application can carefully control its dose, for example by controlling operation of a controlled delivery device such as the controlled delivery device 150 described above in relation to Fig. 2.

In some examples, the medical or surgical apparatus may be configured to deliver a plurality of non-cytotoxic proteases, for example different serotypes of non-cytotoxic protease, such as different serotypes of clostridial neurotoxins, such as BoNTs and/or TeNTs. This may be beneficial, for example, to inhibit host immunogenicity.

In some examples, the medical or surgical apparatus may be configured to deliver different medical compositions, wherein each medical composition may comprise a different polypeptide. Each medical composition (for example polypeptide) may comprise at least one non-cytotoxic protease (in some examples the non-cytotoxic protease may be the same between compositions, but in other examples the compositions may comprise one or more different non-cytotoxic proteases). Each composition, for example each polypeptide, may comprise different targeting moieties and/or different translocation domains. In some examples the different medical compositions, for example different polypeptides, may comprise different TSIs that bind to different receptors on the same target cell.

In some examples, the medical or surgical apparatus may be configured to deliver a plurality of doses. For example, the ultrasound imaging processing application may be configured to deliver a plurality of doses, for example by control of the controlled delivery device 150 of Fig. 2. The plurality of doses may be of the same type of non-cytotoxic protease, or may be of different serotypes, for example to inhibit host immunogenicity.

For example, the ultrasound imaging processing application may comprise a user interface allowing the user to enter details of the desired treatment (such as the serotype(s) of non-cytotoxic protease(s)) and/or details regarding the patient (such as age, weight, clinical symptoms etc.), and the application may be configured to reference the database of treatment sites and dosage regimes to determine a treatment regime for the patient.

In some examples, the ultrasound imaging processing application may also be configured to determine which serotypes (and optionally quantities of each of those serotypes) to administer to the patient (and in some cases the locations of where to inject those serotypes). Information regarding the serotypes and at least one of the treatment regime, doses and sites for each of the serotypes may be provided as part of the treatment guidance.

In some examples, therefore, the ultrasound imaging processing application may be configured to provide treatment guidance that comprises an indication of at least one of:
(i) where to inject each of the plurality of non-cytotoxic proteases relative to the patient being scanned;
(ii) which particular serotype of non-cytotoxic protease to inject at a selected treatment site; and
(iii) a quantity of each of the non-cytotoxic proteases to inject at a selected treatment site.

In some examples, the ultrasound imaging handheld scanner 100 and/or ultrasound imaging application may be calibrated so that the location of an injectable device, for example a controlled delivery device 150 (as described above in relation to Fig. 2) or an injectable device 170 inserted into a guide 160 (as described above in relation to Fig. 3) is determined. Calibration may involve taking a reference scan at a first position and marking the position of the injectable device/controlled delivery device on the patient's skin at the first position. A second scan may then be taken with the ultrasound transducer 106 located on the patient's skin at the first position, so that the distance between the ultrasound transducer 106 and the injectable device can be determined by comparison of the ultrasound data obtained at the first position and the second position.

Figs. 6 to 8 show example screenshots of a computer program operating to provide a dosage application. In the examples shown the computer program is operating a handheld device (in this case a smartphone), although it will be understood that the computer program may be running on other types of device, for example a computer running the ultrasound imaging processing application as described above and/or on a device in communication with a computer running the ultrasound imaging processing application.

Fig. 6 shows two similar screenshots of the computer program operating to provide the dosage application. The dosage application allows a user to select a muscle type and to select a dose for that muscle type. For each muscle type the dosage application shows a recommended dose range for the patient. If a dose outside of the recommended range is selected a warning may be presented to the user. The recommended dose range may be calculated based on information relating to the patient - such as their age and weight. The dosage application also determines the total dose that may be administered to a patient based on the selected doses for each muscle type, and a total approved dose for the patient. If the total dose is above the total approved dose a warning may be provided to the user. The total approved dose may also be calculated based on information relating to the patient - such as their age and weight.

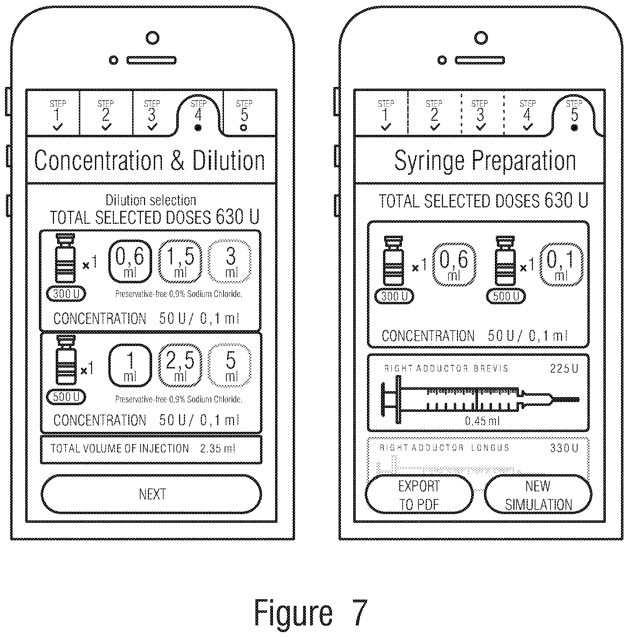

Fig. 7 shows two more similar screenshots of the computer program operating to provide the dosage application. In the screenshots of Fig. 7 it can be seen how the dosage application can determine the volume of injection to be administered based on the desired dose and the concentration of treatment solution that is to be used. The dosage application can therefore display an indication to the user of the volume of injection to be administered to a patient at each treatment site.

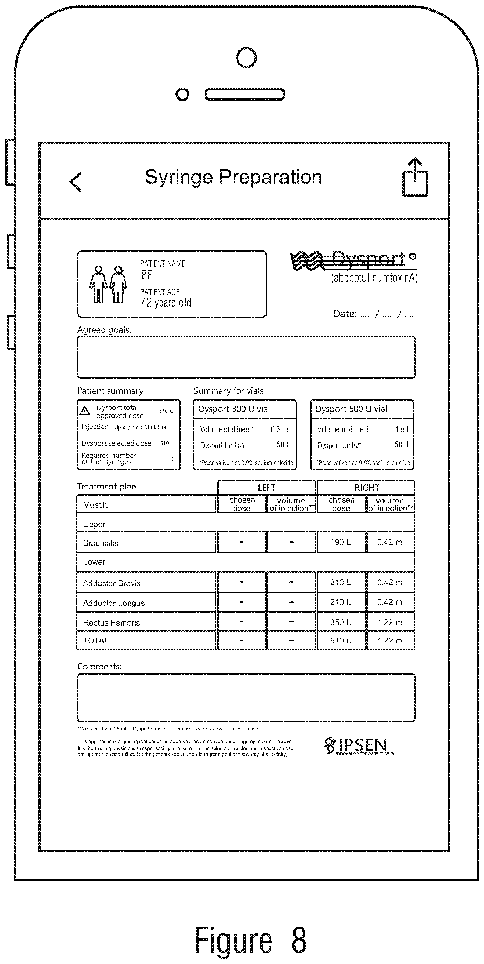

Fig. 8 shows another screenshot of the computer program operating to provide the dosage application. As can be seen in Fig. 8, the dosage application can display an indication of the doses and volumes of treatment solutions to be injected at each treatment site.

### Examples

A region of a patient's anatomy is scanned with an ultrasound imaging handheld scanner (such as the scanner 100 described above in relation to Figs. 1 to 3) to obtain a graphical representation of the region of the patient's anatomy being scanned. A database of treatment sites and dosage regimes is referenced, for example, for the non-cytotoxic protease being administered. At least one of: (i) the correct location to inject the non-cytotoxic protease being administered based on the graphical representation of the region of the patient's anatomy and the reference with the database of treatment sites and dosage regimes; and (ii) the correct dose of the non-cytotoxic protease to administer based on the graphical representation of the region of the patient's anatomy and the reference with the database of treatment sites and dosage regimes is determined. A controlled dose of the non-cytotoxic protease is administered at the correct location based on the determination.

### Example 1

A 45 year-old female patient presents with hemifacial spasm. The patient's face is scanned using the ultrasound imaging handheld scanner 100 connected to a computer running an ultrasound imaging processing application. The ultrasound imaging processing application references a database of treatment sites and dosage regimes for at least one non-cytotoxic protease, and provides treatment guidance for guiding treatment based on the received data from the ultrasound imaging handheld scanner and the database of treatment sites and dosage regimes for the at least one non-cytotoxic protease.

### Example 2

A 55 year-old male patient presents with spasticity of the right arm following from a stroke. The patient's degree of disability due to the spasticity is given a score based on the patient's symptoms. The patient's arm is scanned using the ultrasound imaging handheld scanner 100 connected to a computer running an ultrasound imaging processing application. The ultrasound imaging processing application references a database of treatment sites and dosage regimes for at least one non-cytotoxic protease, and provides treatment guidance for guiding treatment based on the received data from the ultrasound imaging handheld scanner, the database of treatment sites and dosage regimes for the at least one non-cytotoxic protease, and the patient's degree of spasticity and time since the disabilitating event (in this case the stroke).

It will be appreciated from the discussion above that the embodiments shown in the Figures are merely exemplary, and include features which may be generalised, removed or replaced within the scope of the invention, as defined by the appended claims.

## Claims

1. A medical or surgical apparatus for guiding the injection of at least one non-cytotoxic protease, the apparatus comprising:
an ultrasound imaging handheld scanner; and
a computer program comprising instructions which, when the program is executed by a computer, cause the computer to run an ultrasound imaging processing application;
wherein the ultrasound imaging handheld scanner comprises:
at least one ultrasound transducer for transmitting and receiving ultrasound energy;
a processor coupled to the at least one ultrasound transducer and configured to receive ultrasound energy signals from the at least one ultrasound transducer; and
an interface coupled to the processor and configured to send data to the computer running the ultrasound imaging processing application, wherein the data is based on the received ultrasound energy signal from the at least one ultrasound transducer; and
wherein the ultrasound imaging processing application is configured to reference a database of treatment sites and dosage regimes for at least one non-cytotoxic protease, and is configured to provide treatment guidance to a user of the medical or surgical apparatus for guiding treatment based on the received data from the ultrasound imaging handheld scanner and the database of treatment sites and dosage regimes for the at least one non-cytotoxic protease.

2. The medical or surgical apparatus of claim 1, further comprising a controlled delivery device coupled to the ultrasound imaging handheld scanner, wherein the controlled delivery device is configured to deliver a controlled dose of at least one non-cytotoxic protease to a patient being imaged by the ultrasound imaging handheld scanner based on a location relative to the patient determined based on the received ultrasound energy signals.

3. The medical or surgical apparatus of claim 1, further comprising a guide for receiving at least a portion of an injection device, such as a needle, wherein the guide is coupled to the ultrasound imaging handheld scanner, and wherein the computer running the ultrasound imaging processing application is configured to display information to the user for enabling the user to position the guide at a determined position relative to the patient based on the database of treatment sites and dosage regimes for the at least one non-cytotoxic protease, so that the user can inject the non-cytotoxic protease into the patient at the correct treatment site for the selected non-cytotoxic protease being injected.

4. A medical or surgical apparatus for guiding the injection of at least one non-cytotoxic protease, the apparatus comprising:
an ultrasound imaging handheld scanner; and
a controlled delivery device coupled to the ultrasound imaging handheld scanner;
wherein the ultrasound imaging handheld scanner comprises:
at least one ultrasound transducer for transmitting and receiving ultrasound energy;
a processor coupled to the at least one ultrasound transducer and configured to receive ultrasound energy signals from the at least one ultrasound transducer; and
wherein the controlled delivery device is configured to deliver a controlled dose of at least one non-cytotoxic protease to a patient being imaged by the ultrasound imaging handheld scanner based on a location relative to the patient determined based on the received ultrasound energy signal.

5. The medical or surgical apparatus of claim 4 further comprising an interface coupled to the processor and configured to send data to a computer running an ultrasound imaging processing application, wherein the data is based on the received ultrasound energy signals from the at least one ultrasound transducer;
wherein the ultrasound imaging processing application comprises a database of treatment sites and dosage regimes for at least one non-cytotoxic protease, and is configured to provide treatment guidance to a user of the medical or surgical apparatus for guiding treatment based on the received data from the ultrasound imaging handheld scanner and the database of treatment sites and dosage regimes for the at least one non-cytotoxic protease.

6. The medical or surgical apparatus of any of claims 1 to 3 or 5, wherein the treatment guidance comprises an indication of at least one of:
(i) where to inject the non-cytotoxic protease relative to the patient being scanned; and
(ii) a quantity of non-cytotoxic protease to inject; and
optionally wherein the treatment guidance comprises an indication of where to inject the non-cytotoxic protease relative to the patient being scanned, and wherein the indication is overlaid on a graphical representation of the patient being scanned based on the data received from the ultrasound imaging handheld scanner.

7. The medical or surgical apparatus of claim 2, 3 or 5, wherein the ultrasound imaging processing application is configured to determine the position of the controlled delivery device, or an injection device positioned in the guide, relative to the patient based on the received data from the ultrasound imaging handheld device, and wherein the treatment guidance comprises an indication to the user of how to position the ultrasound imaging handheld scanner with respect to the patient so that the non-cytotoxic protease can be injected from the controlled delivery device or injection device positioned in the guide at the correct treatment site.

8. The medical or surgical apparatus of any of claims 1 to 3 or 5 to 7 wherein the ultrasound imaging processing application is configured to process the data received from the ultrasound imaging handheld device to provide a graphical representation of the patient being scanned to the user.

9. The medical or surgical apparatus of any of claims 1 to 7 wherein the processor of the ultrasound imaging handheld scanner is configured to process the received ultrasound energy signals from the at least one ultrasound transducer to provide data signals comprising information indicative of a graphical representation of the patient being scanned.

10. A medical or surgical kit comprising:
an ultrasound imaging handheld scanner;
an injectable device comprising a selected quantity of at least one non-cytotoxic protease; and
a computer program comprising instructions which, when the program is executed by a computer, cause the computer to run an ultrasound imaging processing application; wherein the ultrasound imaging processing application is configured to reference a database of treatment sites and dosage regimes for at least one non-cytotoxic protease, and is configured to provide treatment guidance to a user of the medical or surgical apparatus for guiding treatment based on the received data from the ultrasound imaging handheld scanner and the database of treatment sites and dosage regimes for the at least one non-cytotoxic protease, for enabling the user to inject the correct quantity of non-cytotoxic protease at the correct treatment site;
optionally further comprising at least one of
(i) a controlled delivery device coupled to the ultrasound imaging handheld scanner, wherein the controlled delivery device is configured to deliver a controlled dose of at least one non-cytotoxic protease to a patient being imaged by the ultrasound imaging handheld scanner based on a location relative to the patient determined based on the received ultrasound energy signal, and
(ii) a guide for receiving at least a portion of an injection device, such as a needle, wherein the guide is coupled to the ultrasound imaging handheld scanner, and wherein the computer running the ultrasound imaging processing application is configured to display information to the user for enabling the user to position the guide at a determined position relative to the patient based on the database of treatment sites and dosage regimes for the at least one non-cytotoxic protease, so that the user can inject the non-cytotoxic protease into the patient at the correct treatment site for the selected non-cytotoxic protease being injected.

11. A computer program comprising instructions which, when the program is executed by a computer, cause the computer to run an ultrasound imaging processing application, wherein the ultrasound imaging processing application is configured to:
receive and process data obtained from an ultrasound imaging handheld scanner;
reference a database of treatment sites and dosage regimes for at least one non-cytotoxic protease; and
provide treatment guidance to a user of the medical or surgical apparatus for guiding treatment based on the received data from the ultrasound transducer and the database of treatment sites and dosage regimes for the at least one non-cytotoxic protease, for enabling the user to inject the correct quantity of non-cytotoxic protease at the correct treatment site.

12. The computer program of claim 11 wherein the ultrasound imaging processing application is configured to process the data received from the ultrasound imaging handheld device to provide a graphical representation of the patient being scanned to the user.

13. The computer program of claim 11 or 12 wherein the treatment guidance comprises an indication of where to inject the non-cytotoxic protease relative to the patient being scanned, and wherein the indication is overlaid on a graphical representation of the patient being scanned based on the data received from the ultrasound imaging handheld scanner.

14. The computer program of any of claims 11 to 13 wherein the ultrasound imaging processing application is configured to determine the position of at least one of:
(i) a controlled delivery device coupled to the handheld ultrasound imaging scanner, and
(ii) an injection device positioned in a guide and coupled to the handheld ultrasound imaging scanner, relative to the patient based on the received data from the ultrasound imaging handheld device, and
wherein the treatment guidance comprises an indication to the user of how to position the ultrasound imaging handheld scanner with respect to the patient so that the non-cytotoxic protease can be injected from the controlled delivery device or injection device positioned in the guide at the correct treatment site.

## Patentansprüche

1. Medizinische oder chirurgische Apparatur zum Anleiten der Injektion mindestens einer nichtzytotoxischen Protease, wobei die Apparatur umfasst:
einen Handultraschallabbildungsscanner und
ein Computerprogramm, das Anweisungen umfasst, die, wenn das Programm von einem Computer ausgeführt wird, bewirken, dass der Computer eine Ultraschallabbildungsverarbeitungsanwendung ausführt;
wobei der Handultraschallabbildungsscanner umfasst:
mindestens einen Ultraschallwandler zum Übertragen und Empfangen von Ultraschallenergie;
einen Prozessor, der an den mindestens einen Ultraschallwandler gekoppelt ist und dazu konfiguriert ist, Ultraschallenergiesignale von dem mindestens einen Ultraschallwandler zu empfangen; und
eine Schnittstelle, die an den Prozessor gekoppelt ist und dazu konfiguriert ist, Daten an den Computer, der die Ultraschallabbildungsverarbeitungsanwendung ausführt, zu senden, wobei die Daten auf dem empfangenen Ultraschallenergiesignal von dem mindestens einen Ultraschallwandler basieren; und
wobei die Ultraschallabbildungsverarbeitungsanwendung dazu konfiguriert ist, sich auf eine Datenbank von Behandlungsorten und Dosierungsplänen für mindestens eine nichtzytotoxische Protease zu beziehen, und dazu konfiguriert ist, einem Benutzer der medizinischen oder chirurgischen Apparatur eine Behandlungsanleitung zum Anleiten einer Behandlung auf der Basis der empfangenen Daten von dem Handultraschallabbildungsscanner und der Datenbank von Behandlungsorten und Dosierungsplänen für die mindestens eine nichtzytotoxische Protease bereitzustellen.

2. Medizinische oder chirurgische Apparatur nach Anspruch 1, weiterhin umfassend eine Vorrichtung zur kontrollierten Abgabe, die an den Handultraschallabbildungsscanner gekoppelt ist, wobei die Vorrichtung zur kontrollierten Abgabe dazu konfiguriert ist, eine kontrollierte Dosis mindestens einer nichtzytotoxischen Protease an einen Patienten, der durch den Handultraschallabbildungsscanner abgebildet wird, auf der Basis einer Stelle relativ zu dem Patienten, die auf der Basis der empfangenen Ultraschallenergiesignale bestimmt wird, abzugeben.

3. Medizinische oder chirurgische Apparatur nach Anspruch 1, weiterhin umfassend eine Führung zum Aufnehmen mindestens eines Teils einer Injektionsvorrichtung, wie einer Nadel, wobei die Führung an den Handultraschallabbildungsscanner gekoppelt ist, und wobei der Computer, der die Ultraschallabbildungsverarbeitungsanwendung ausführt, dazu konfiguriert ist, dem Benutzer Informationen anzuzeigen, um dem Benutzer zu ermöglichen, die Führung in einer bestimmten Position relativ zu dem Patienten auf der Basis der Datenbank von Behandlungsorten und Dosierungsplänen für die mindestens eine nichtzytotoxische Protease zu positionieren, so dass der Benutzer die nichtzytotoxische Protease in den Patienten am korrekten Behandlungsort für die ausgewählte nichtzytotoxische Protease, die injiziert wird, injizieren kann.

4. Medizinische oder chirurgische Apparatur zum Anleiten der Injektion mindestens einer nichtzytotoxischen Protease, wobei die Apparatur umfasst:
einen Handultraschallabbildungsscanner und
eine Vorrichtung zur kontrollierten Abgabe, die an den Handultraschallabbildungsscanner gekoppelt ist;
wobei der Handultraschallabbildungsscanner umfasst:
mindestens einen Ultraschallwandler zum Übertragen und Empfangen von Ultraschallenergie;
einen Prozessor, der an den mindestens einen Ultraschallwandler gekoppelt ist und dazu konfiguriert ist, Ultraschallenergiesignale von dem mindestens einen Ultraschallwandler zu empfangen; und
wobei die Vorrichtung zur kontrollierten Abgabe dazu konfiguriert ist, eine kontrollierte Dosis mindestens einer nichtzytotoxischen Protease an einen Patienten, der durch den Handultraschallabbildungsscanner abgebildet wird, auf der Basis einer Stelle relativ zu dem Patienten, die auf der Basis des empfangenen Ultraschallenergiesignals bestimmt wird, abzugeben.

5. Medizinische oder chirurgische Apparatur nach Anspruch 4, weiterhin umfassend eine Schnittstelle, die an den Prozessor gekoppelt ist und dazu konfiguriert ist, Daten an einen Computer, der eine Ultraschallabbildungsverarbeitungsanwendung ausführt, zu senden, wobei die Daten auf den empfangenen Ultraschallenergiesignalen von dem mindestens einen Ultraschallwandler basieren;
wobei die Ultraschallabbildungsverarbeitungsanwendung eine Datenbank von Behandlungsorten und Dosierungsplänen für mindestens eine nichtzytotoxische Protease umfasst und dazu konfiguriert ist, einem Benutzer der medizinischen oder chirurgischen Apparatur eine Behandlungsanleitung zum Anleiten einer Behandlung auf der Basis der empfangenen Daten von dem Handultraschallabbildungsscanner und der Datenbank von Behandlungsorten und Dosierungsplänen für die mindestens eine nichtzytotoxische Protease bereitzustellen.

6. Medizinische oder chirurgische Apparatur nach einem der Ansprüche 1 bis 3 oder 5, wobei die Behandlungsanleitung eine Angabe mindestens eines der folgenden umfasst:
(i) wo die nichtzytotoxische Protease relativ zu dem Patienten, der gescannt wird, zu injizieren ist; und
(ii) eine Menge einer zu injizierenden nichtzytotoxischen Protease; und
optional wobei die Behandlungsanleitung eine Angabe umfasst, wo die nichtzytotoxische Protease relativ zu dem Patienten, der gescannt wird, zu injizieren ist, und wobei die Angabe auf eine graphische Darstellung des Patienten, der gescannt wird, auf der Basis der Daten, die von dem Handultraschallabbildungsscanner empfangen werden, überlagert ist.

7. Medizinische oder chirurgische Apparatur nach Anspruch 2, 3 oder 5, wobei die Ultraschallabbildungsverarbeitungsanwendung dazu konfiguriert ist, die Position der Vorrichtung zur kontrollierten Abgabe oder einer Injektionsvorrichtung, die in der Führung positioniert ist, relativ zu dem Patienten auf der Basis der empfangenen Daten von der Handultraschallabbildungsvorrichtung zu bestimmen, und wobei die Behandlungsanleitung eine Angabe an den Benutzer umfasst, wie der Handultraschallabbildungsscanner in Bezug auf den Patienten zu positionieren ist, so dass die nichtzytotoxische Protease aus der Vorrichtung zur kontrollierten Abgabe oder der Injektionsvorrichtung, die in der Führung positioniert ist, am korrekten Behandlungsort injiziert werden kann.

8. Medizinische oder chirurgische Apparatur nach einem der Ansprüche 1 bis 3 oder 5 bis 7, wobei die Ultraschallabbildungsverarbeitungsanwendung dazu konfiguriert ist, die Daten, die von der Handultraschallabbildungsvorrichtung empfangen werden, zu verarbeiten, um dem Benutzer eine graphische Darstellung des Patienten, der gescannt wird, bereitzustellen.

9. Medizinische oder chirurgische Apparatur nach einem der Ansprüche 1 bis 7, wobei der Prozessor des Handultraschallabbildungsscanners dazu konfiguriert ist, die empfangenen Ultraschallenergiesignale von dem mindestens einen Ultraschallwandler zu verarbeiten, um Datensignale bereitzustellen, die Informationen umfassen, die eine graphische Darstellung des Patienten, der gescannt wird, angeben.

10. Medizinisches oder chirurgisches Kit, umfassend:
einen Handultraschallabbildungsscanner;
eine injizierbare Vorrichtung, die eine ausgewählte Menge mindestens einer nichtzytotoxischen Protease umfasst; und
ein Computerprogramm, das Anweisungen umfasst, die, wenn das Programm von einem Computer ausgeführt wird, bewirken, dass der Computer eine Ultraschallabbildungsverarbeitungsanwendung ausführt;
wobei die Ultraschallabbildungsverarbeitungsanwendung dazu konfiguriert ist, sich auf eine Datenbank von Behandlungsorten und Dosierungsplänen für mindestens eine nichtzytotoxische Protease zu beziehen, und dazu konfiguriert ist, einem Benutzer der medizinischen oder chirurgischen Apparatur eine Behandlungsanleitung zum Anleiten einer Behandlung auf der Basis der empfangenen Daten von dem Handultraschallabbildungsscanner und der Datenbank von Behandlungsorten und Dosierungsplänen für die mindestens eine nichtzytotoxische Protease bereitzustellen, um dem Benutzer zu ermöglichen, die korrekte Menge einer nichtzytotoxischen Protease am korrekten Behandlungsort zu injizieren;
optional weiterhin umfassend mindestens eine von:
(i) einer Vorrichtung zur kontrollierten Abgabe, die an den Handultraschallabbildungsscanner gekoppelt ist, wobei die Vorrichtung zur kontrollierten Abgabe dazu konfiguriert ist, eine kontrollierte Dosis mindestens einer nichtzytotoxischen Protease an einen Patienten, der durch den Handultraschallabbildungsscanner abgebildet wird, auf der Basis einer Stelle relativ zu dem Patienten, die auf der Basis des empfangenen Ultraschallenergiesignals bestimmt wird, abzugeben, und
(ii) einer Führung zum Aufnehmen mindestens eines Teils einer Injektionsvorrichtung, wie einer Nadel, wobei die Führung an den Handultraschallabbildungsscanner gekoppelt ist, und wobei der Computer, der die Ultraschallabbildungsverarbeitungsanwendung ausführt, dazu konfiguriert ist, dem Benutzer Informationen anzuzeigen, um dem Benutzer zu ermöglichen, die Führung in einer bestimmten Position relativ zu dem Patienten auf der Basis der Datenbank von Behandlungsorten und Dosierungsplänen für die mindestens eine nichtzytotoxische Protease zu positionieren, so dass der Benutzer die nichtzytotoxische Protease in den Patienten am korrekten Behandlungsort für die ausgewählte nichtzytotoxische Protease, die injiziert wird, injizieren kann.

11. Computerprogramm, das Anweisungen umfasst, die, wenn das Programm von einem Computer ausgeführt wird, bewirken, dass der Computer eine Ultraschallabbildungsverarbeitungsanwendung ausführt, wobei die Ultraschallabbildungsverarbeitungsanwendung konfiguriert ist zum:
Empfangen und Verarbeiten von Daten, die von einem Handultraschallabbildungsscanner erhalten werden;
Beziehen auf eine Datenbank von Behandlungsorten und Dosierungsplänen für mindestens eine nichtzytotoxische Protease und
Bereitstellen einer Behandlungsanleitung an einen Benutzer der medizinischen oder chirurgischen Apparatur zum Anleiten einer Behandlung auf der Basis der empfangenen Daten von dem Ultraschallwandler und der Datenbank von Behandlungsorten und Dosierungsplänen für die mindestens eine nichtzytotoxische Protease bereitzustellen, um dem Benutzer zu ermöglichen, die korrekte Menge einer nichtzytotoxischen Protease am korrekten Behandlungsort zu injizieren.

12. Computerprogramm nach Anspruch 11, wobei die Ultraschallabbildungsverarbeitungsanwendung dazu konfiguriert ist, die Daten, die von der Handultraschallabbildungsvorrichtung empfangen werden, zu verarbeiten, um dem Benutzer eine graphische Darstellung des Patienten, der gescannt wird, bereitzustellen.

13. Computerprogramm nach Anspruch 11 oder 12, wobei die Behandlungsanleitung eine Angabe umfasst, wo die nichtzytotoxische Protease relativ zu dem Patienten, der gescannt wird, zu injizieren ist, und wobei die Angabe auf eine graphische Darstellung des Patienten, der gescannt wird, auf der Basis der Daten, die von dem Handultraschallabbildungsscanner empfangen werden, überlagert ist.

14. Computerprogramm nach einem der Ansprüche 11 bis 13, wobei die Ultraschallabbildungsverarbeitungsanwendung dazu konfiguriert ist, die Position mindestens einer der folgenden:
(i) einer Vorrichtung zur kontrollierten Abgabe, die an den Handultraschallabbildungsscanner gekoppelt ist, und
(ii) einer Injektionsvorrichtung, die in einer Führung positioniert ist und an einen Handultraschallabbildungsscanner gekoppelt ist, relativ zu dem Patienten auf der Basis der empfangenen Daten von der Handultraschallabbildungsvorrichtung zu bestimmen, und
wobei die Behandlungsanleitung eine Angabe an den Benutzer umfasst, wie der Handultraschallabbildungsscanner in Bezug auf den Patienten zu positionieren ist, so dass die nichtzytotoxische Protease aus der Vorrichtung zur kontrollierten Abgabe oder der Injektionsvorrichtung, die in der Führung positioniert ist, am korrekten Behandlungsort injiziert werden kann.

## Revendications

1. Appareil médical ou chirurgical destiné à guider l'injection d'au moins une protéase non cytotoxique, l'appareil comprenant :
un appareil de balayage à main d'imagerie par ultrasons ; et
un programme d'ordinateur comprenant des instructions qui, quand le programme est exécuté par un ordinateur, amènent l'ordinateur à exécuter une application de traitement d'imagerie par ultrasons ;
dans lequel l'appareil de balayage à main d'imagerie par ultrasons comprend :
au moins un transducteur à ultrasons destiné à émettre et à recevoir de l'énergie ultrasonore ;
un processeur couplé à l'au moins un transducteur à ultrasons et configuré pour recevoir des signaux d'énergie ultrasonore de l'au moins un transducteur à ultrasons ; et
une interface couplée au processeur et configurée pour envoyer des données à l'ordinateur exécutant l'application de traitement d'imagerie par ultrasons, dans lequel les données sont basées sur le signal d'énergie ultrasonore reçu de l'au moins un transducteur à ultrasons ; et
dans lequel l'application de traitement d'imagerie par ultrasons est configurée pour référencer une base de données de sites de traitement et de schémas posologiques pour au moins une protéase non cytotoxique, et est configuré pour fournir des recommandations en matière de traitement à un utilisateur de l'appareil médical ou chirurgical destinées à guider le traitement sur la base des données reçues de l'appareil de balayage à main d'imagerie par ultrasons et de la base données de sites de traitement et de schémas posologiques pour l'au moins une protéase non cytotoxique.

2. Appareil médical ou chirurgical selon la revendication 1, comprenant en outre un dispositif de délivrance contrôlée couplé à l'appareil de balayage à main d'imagerie par ultrasons, dans lequel le dispositif de délivrance contrôlée est configuré pour délivrer une dose contrôlée d'au moins une protéase non cytotoxique à un patient en cours d'imagerie par l'appareil de balayage à main d'imagerie par ultrasons sur la base d'un emplacement par rapport au patient déterminé sur la base des signaux d'énergie ultrasonores reçus.

3. Appareil médical ou chirurgical selon la revendication 1, comprenant en outre un guide destiné à recevoir au moins une partie d'un dispositif d'injection, tel qu'une aiguille, dans lequel le guide est couplé à l'appareil de balayage à main d'imagerie par ultrasons, et dans lequel l'ordinateur exécutant l'application de traitement d'imagerie par ultrasons est configuré pour afficher des informations à l'attention de l'utilisateur destinées à permettre à l'utilisateur de positionner le guide au niveau d'une position déterminée par rapport au patient sur la base de la base de données de sites de traitement et de schémas posologiques pour l'au moins une protéase non cytotoxique, de sorte que l'utilisateur peut injecter la protéase non cytotoxique dans le patient au niveau du bon site de traitement pour la protéase non cytotoxique sélectionnée étant injectée.

4. Appareil médical ou chirurgical destiné à guider l'injection d'au moins une protéase non cytotoxique, l'appareil comprenant :
un appareil de balayage à main d'imagerie par ultrasons ; et
un dispositif de délivrance contrôlée couplé à l'appareil de balayage à main d'imagerie par ultrasons ;
dans lequel l'appareil de balayage à main d'imagerie par ultrasons comprend :
au moins un transducteur à ultrasons destiné à émettre et à recevoir de l'énergie ultrasonore ;
un processeur couplé à l'au moins un transducteur à ultrasons et configuré pour recevoir des signaux d'énergie ultrasonore de l'au moins un transducteur à ultrasons ; et
dans lequel le dispositif de délivrance contrôlée est configuré pour délivrer une dose contrôlée d'au moins une protéase non cytotoxique à un patient en cours d'imagerie par l'appareil de balayage à main d'imagerie par ultrasons sur la base d'un emplacement par rapport au patient déterminé sur la base du signal d'énergie ultrasonore reçu.

5. Appareil médical ou chirurgical selon la revendication 4 comprenant en outre une interface couplée au processeur et configurée pour envoyer des données à un ordinateur exploitant une application de traitement d'imagerie par ultrasons, dans lequel les données sont basées sur les signaux d'énergie ultrasonore reçus de l'au moins un transducteur à ultrasons ;
dans lequel l'application de traitement d'imagerie par ultrasons comprend une base de données de sites de traitement et de schémas posologiques pour au moins une protéase non-cytotoxique, et est configurée pour fournir des recommandations en matière de traitement à un utilisateur de l'appareil médical ou chirurgical destinées à guider le traitement sur la base des données reçues de l'appareil de balayage à main d'imagerie par ultrasons et de la base de données de sites de traitement et de schémas posologiques pour l'au moins une protéase non cytotoxique.

6. Appareil médical ou chirurgical selon l'une quelconque des revendications 1 à 3 ou 5, dans lequel les recommandations en matière de traitement comprennent une indication d'au moins un élément parmi :
(i) l'endroit où il faut injecter de la protéase non cytotoxique par rapport au patient étant soumis au balayage ; et
(ii) une quantité de protéase non cytotoxique qu'il faut injecter ; et
facultativement dans lequel les recommandations en matière de traitement comprennent une indication de l'endroit où il faut injecter la protéase non cytotoxique par rapport au patient étant soumis au balayage, et dans lequel l'indication est superposée sur une représentation graphique du patient étant soumis au balayage sur la base des données reçues de l'appareil de balayage à main d'imagerie par ultrasons.

7. Appareil médical ou chirurgical selon la revendication 2, 3 ou 5, dans lequel l'application de traitement d'imagerie par ultrasons est configurée pour déterminer la position du dispositif de délivrance contrôlée, ou d'un dispositif d'injection positionné dans le guide, par rapport au patient sur la base des données reçues du dispositif à main d'imagerie par ultrasons, et dans lequel les recommandations en matière de traitement comprennent une indication à l'utilisateur de la manière dont il faut positionner l'appareil de balayage à main d'imagerie par ultrasons par rapport au patient de sorte que la protéase non cytotoxique peut être injectée depuis le dispositif de délivrance contrôlée ou le dispositif d'injection positionné dans le guide au niveau du bon site de traitement.

8. Appareil médical ou chirurgical selon l'une quelconque des revendications 1 à 3 ou 5 à 7 dans lequel l'application de traitement d'imagerie par ultrasons est configurée pour traiter les données reçues du dispositif à main d'imagerie par ultrasons pour fournir une représentation graphique du patient étant soumis au balayage à l'utilisateur.

9. Appareil médical ou chirurgical selon l'une quelconque des revendications 1 à 7 dans lequel le processeur de l'appareil de balayage à main d'imagerie par ultrasons est configuré pour traiter les signaux d'énergie ultrasonore reçus de l'au moins un transducteur à ultrasons pour fournir des signaux de données comprenant des informations indiquant une représentation graphique du patient étant soumis au balayage.

10. Trousse médicale ou chirurgicale comprenant :
un appareil de balayage à main d'imagerie par ultrasons ;
un dispositif injectable comprenant une quantité sélectionnée d'au moins une protéase non cytotoxique ; et
un programme d'ordinateur comprenant des instructions qui, quand le programme est exécuté par ordinateur, amènent l'ordinateur à exécuter une application de traitement d'imagerie par ultrasons ;
dans lequel l'application de traitement d'imagerie par ultrasons est configurée pour référencer une base de données de sites de traitement et de schémas posologiques pour au moins une protéase non cytotoxique, et est configurée pour fournir des recommandations en matière de traitement à un utilisateur de l'appareil médical ou chirurgical destinées à guider le traitement sur la base des données reçues de l'appareil de balayage à main d'imagerie par ultrasons et de la base données de sites de traitement et de schémas posologiques pour l'au moins une protéase non cytotoxique, destinées à permettre à l'utilisateur d'injecter la bonne quantité de protéase non cytotoxique au niveau du bon site de traitement ;
comprenant en outre facultativement au moins un élément parmi :
(i) un dispositif de délivrance contrôlée couplé à l'appareil de balayage à main d'imagerie par ultrasons, dans lequel le dispositif de délivrance contrôlée est configuré pour délivrer une dose contrôlée d'au moins une protéase non cytotoxique à un patient en cours d'imagerie par l'appareil de balayage à main d'imagerie par ultrasons sur la base d'un emplacement par rapport au patient déterminé sur la base du signal d'énergie ultrasonore reçu, et
(ii) un guide destiné à recevoir au moins une partie d'un dispositif d'injection, telle qu'une aiguille, dans lequel le guide est couplé à l'appareil de balayage à main d'imagerie par ultrasons, et dans lequel l'ordinateur exploitant l'application de traitement d'imagerie par ultrasons est configuré pour afficher des informations à l'attention de l'utilisateur destinées à permettre à l'utilisateur de positionner le guide au niveau d'une position déterminée par rapport au patient sur la base de la base de données de sites de traitement et de schémas posologiques pour l'au moins une protéase non cytotoxique, de sorte que l'utilisateur peut injecter la protéase non cytotoxique dans le patient au niveau du bon site de traitement pour la protéase non cytotoxique sélectionnée étant injectée.

11. Programme d'ordinateur comprenant des instructions qui, quand le programme est exécuté par un ordinateur, amènent l'ordinateur à exploiter une application de traitement d'imagerie par ultrasons, dans lequel l'application de traitement d'imagerie par ultrasons est configurée pour :
recevoir et traiter des données obtenues à partir d'un appareil de balayage à main d'imagerie par ultrasons ;
référencer une base de données de sites de traitement et de schémas posologiques pour au moins une protéase non cytotoxique ; et
fournir des recommandations en matière de traitement à un utilisateur de l'appareil médical ou chirurgical destinées à guider le traitement sur la base des données reçues du transducteur à ultrasons et de la base de données des sites de traitement et des schémas posologiques pour l'au moins une protéase non cytotoxique, destinées à permettre à l'utilisateur d'injecter la bonne quantité de protéase non cytotoxique au niveau du bon site de traitement.

12. Programme d'ordinateur selon la revendication 11 dans lequel l'application de traitement d'imagerie par ultrasons est configurée pour traiter les données reçues du dispositif à main d'imagerie par ultrasons pour fournir une représentation graphique du patient soumis au balayage à l'utilisateur.

13. Programme d'ordinateur selon la revendication 11 ou 12 dans lequel les recommandations en matière de traitement comprennent une indication de l'endroit où il faut injecter la protéase non cytotoxique par rapport au patient soumis au balayage, et dans lequel l'indication est superposée sur une représentation graphique du patient soumis au balayage sur la base des données reçues de l'appareil de balayage à main d'imagerie par ultrasons.

14. Programme d'ordinateur selon l'une quelconque des revendication 11 à 13 dans lequel l'application de traitement d'imagerie par ultrasons est configurée pour déterminer la position d'au moins un élément parmi :
(i) un dispositif de délivrance contrôlée couplé à l'appareil de balayage à main d'imagerie par ultrasons, et
(ii) un dispositif d'injection positionné dans un guide et couplé à l'appareil de balayage à main d'imagerie par ultrasons, par rapport au patient sur la base des données reçues du dispositif à main d'imagerie par ultrasons, et
dans lequel les recommandations de traitement comprennent une indication à l'utilisateur de la manière dont il faut positionner l'appareil de balayage à main d'imagerie par ultrasons par rapport au patient de sorte que la protéase non cytotoxique peut être injectée depuis le dispositif de délivrance contrôlée ou le dispositif d'injection positionné dans le guide au niveau du bon site de traitement.
